# EUROPÄISCHE PATENTSCHRIFT

(11) **EP 4 277 549 B1**
(45) Veröffentlichungstag und Bekanntmachung des Hinweises auf die Patenterteilung: **08.04.2026**
(21) Anmeldenummer: 22701880.1
(22) Anmeldetag: 11.01.2022
(51) Int. Cl.: A61B 17/221

(54) **THROMBEKTOMIEVORRICHTUNG**
THROMBECTOMY DEVICE
DISPOSITIF DE THROMBECTOMIE

(30) Priorität: 14.01.2021 DE 102021100703
(43) Veröffentlichungstag der Anmeldung: 22.11.2023
(73) Patentinhaber: Phenox GmbH, 44801 Bochum (DE)
(72) Erfinder: HANNES, Ralf, 44137 Dortmund (DE); TRÖSKEN, Volker, 58456 Witten (DE); MONSTADT, Hermann, 44797 Bochum (DE); HENKES, Hans, 70192 Stuttgart (DE)
(74) Vertreter: Schneiders & Behrendt Bochum
(86) Internationale Anmeldenummer: PCT/EP2022/050381
(87) Internationale Veröffentlichungsnummer: WO 2022/152665

(56) Entgegenhaltungen:
- EP-B1- 2 600 795
- KR-A- 20200 133 754
- US-A1- 2007 191 924
- US-A1- 2015 289 892
- US-B1- 9 669 113
- US-B2- 10 292 803

## Beschreibung

Die Erfindung betrifft eine Thrombektomievorrichtung mit einer verbesserten Markierung des proximalen Bereichs. Die Thrombektomievorrichtung umfasst eine im Wesentlichen zylindrische Struktur, deren proximales Ende über Verbindungsstreben mit einem Kopplungselement verbunden ist. Die Thrombektomievorrichtung ist insbesondere dafür bestimmt, Thromben im zerebralen Gefäßsystem zu entfernen.

Thromboembolische Erkrankungen wie Herzinfarkt, Lungenembolie, periphere Thrombose, Organembolien, etc. werden typischerweise durch einen Thromboembolus (im Folgenden kurz: Thrombus), also einen viskoelastischen Blutklumpen aus Thrombozyten, Fibrinogen, Gerinnungsfaktoren etc., ausgelöst, der sich in einem Blutgefäß festgesetzt hat und dieses ganz oder teilweise verschließt. Der Verschluss von Organarterien führt dabei zu einer Unterbrechung der Versorgung des abhängigen Gewebes mit Sauerstoff und Nährstoffen. Der Störung des Funktionsstoffwechsels mit Funktionsverlust folgt innerhalb kurzer Zeit das Erliegen des Strukturstoffwechsels mit dem Untergang des betroffenen Gewebes (Infarkt). Die häufigsten hiervon beim Menschen betroffenen Organe sind das Herz und das Gehirn. Solche Veränderungen betreffen aber auch die Extremitätenarterien und die Lungenarterien. Venöse Thrombosen und thromboembolische Verschlüsse kommen auch gehäuft in den Bein- und Beckenvenen vor. Das Krankheitsbild eines thrombotischen Verschlusses eines intrakraniellen Sinus kann durch die Störung der venösen Drainage des Hirngewebes zu schweren Hirnblutungen führen.

Angesichts der Schwere der durch Thromboembolien ausgelösten Krankheitsbilder und der Häufigkeit dieser Erkrankungen sind verschiedene Techniken zur Auflösung oder Entfernung von Thromben bekannt.

Neben der Behandlung mit thrombolytischen Mitteln zur Auflösung des Thrombus einerseits und offenen chirurgischen Eingriffen zur Entfernung des Thrombus andererseits kommen zunehmend mikroinvasive endovaskuläre Therapieformen zum Einsatz.

Eine dieser bekannten Behandlungsformen betrifft den Einsatz einer sogenannten Thrombektomievorrichtung. Die WO 2012/156069 A1 beschreibt eine solche Thrombektomievorrichtung mit einer zylindrischen Struktur mit einer Vielzahl von Maschen, wie sie auch vaskuläre Stents aufweisen. Die zylindrische Struktur, die im Prinzip einer bekannten Stentstruktur entspricht, umfasst zwei Verbindungsstreben, die in einem Kopplungselement münden und über das Kopplungselement mit einem Einführdraht verbunden sind, der eine präzise Platzierung der Thrombektomievorrichtung am Behandlungsort erlaubt. Das Kopplungselement ist am proximalen Ende der Vorrichtung angeordnet und verbindet dieses mit dem distalen Ende des Einführdrahts. US10292803 B2, US9669113 B1, KR 20200133754 A, US2015/289892 A, US2007/191924 A1 und EP2600795 B1 offenbaren Geräte des Standes der Technik.

Die bekannte Thrombektomievorrichtung weist einen Schlitz auf, der sich wendel- oder helixförmig über die Mantelfläche der zylindrischen, rohrförmigen Struktur erstreckt. Eine solche Thrombektomievorrichtung kann insbesondere durch Laserschneiden aus einem Rohr gefertigt werden, möglich sind jedoch alternative Herstellungsverfahren, beispielsweise über miteinander verflochtene Filamente oder Drähte.

Die Mantelfläche der zylindrischen Struktur bleibt im Bereich des Schlitzes geöffnet, wodurch sich der Durchmesser der zylindrischen Struktur in gewissem Maße an den Durchmesser am Behandlungsort, dem Lumen des Gefäßes, anpassen kann.

Um die zylindrische Struktur mit Schlitz zum einen räumlich zu fixieren und andererseits mit einer gewissen Spannung zu versehen, erstreckt sich am proximalen Ende der zylindrischen Struktur ein Spannbügel bzw. eine Spannstrebe über den Schlitz. Auf diese Weise wird eine insgesamt über den Umfang geschlossene Struktur herbeigeführt, die am proximalen Ende geschlossen ist, während die zylindrische Struktur weiter distal einen helikalen Schlitz aufweist. Der Spannbügel erhöht die Radialkraft der zylindrischen Struktur im proximalen Bereich und dient auch dazu, die einander gegenüberliegenden Kanten des Schlitzes in ihrer Position festzuhalten.

Sofern vorliegend im Zusammenhang mit der Erfindung der Begriff "proximal" gebraucht wird, so bezeichnet dieser Begriff das Ende der Vorrichtung, das beim Einführen der Vorrichtung in den Körper des Patienten zu der behandelnden Person hinweist. Der Begriff "distal" hingegen bezeichnet das von der behandelnden Person wegweisende Ende der Vorrichtung.

Der proximale Bereich der bekannten Thrombektomievorrichtung mit Schlitz bildet entsprechend von dem Spannbügel über Streben der proximalen Maschen und die Verbindungsstreben hin zum Kopplungselement eine geschlossene ringförmige Struktur aus. Auch ohne Schlitz lässt sich eine solche geschlossene ringförmige Struktur am proximalen Ende erreichen; in diesem Fall benötigt man allerdings keinen den Schlitz überspannenden Spannbügel. Die ringförmige Struktur verläuft dabei gegenüber der Längsachse der zylindrischen Struktur geneigt bzw. schräg, mit anderen Worten die (näherungsweise) Ebene, die von der ringförmigen Struktur ausgebildet wird, liegt diagonal zur Längsachse der zylindrischen Struktur und hat eine Erstreckung von proximal nach distal.

Für eine bestmögliche Funktionalität der Thrombektomievorrichtung ist es in jedem Fall von größter Wichtigkeit, dass sich die ringförmige Struktur am Behandlungsort ausreichend weit öffnet und sich auch während des Zurückziehens der Vorrichtung nicht oder zumindest nicht unverhältnismäßig weit zusammenzieht.

Nur mit einem ausreichend weit geöffneten proximalen Ende ist ein erfolgreiches Abtragen des Thrombus mit der Vorrichtung möglich. Entsprechend wäre eine Vorrichtung wünschenswert, die es dem Behandelnden während der Intervention ermöglicht, den dreidimensionalen Zustand der proximalen ringförmigen Struktur zu bewerten. Eine entsprechende Bewertung ist sowohl beim Einbringen der Thrombektomievorrichtung als auch beim Zurückziehen der Thrombektomievorrichtung von entscheidender Bedeutung für den Behandlungserfolg.

Eine Hilfe bei der Bewertung während der Intervention können röntgensichtbare Marker beziehungsweise röntgendichte Markierungen sein. Solche Marker sind im Bereich der medizinischen Implantate und Vorrichtungen, beispielsweise von Stents und Katheterballonen, seit langer Zeit bekannt. Entsprechende Marker sind in der Regel punktuell angebracht und markieren beispielsweise das proximale und distale Ende eines Stents oder Katheters oder auch besondere Bereiche eines Stents oder Katheters wie die Fenestrierung im Fall von Bifurkationsimplantaten.

Die bekannten Marker sind für die notwendige Beurteilung des Öffnungsverhaltens beziehungsweise der Öffnung der proximalen ringförmigen Struktur der Thrombektomievorrichtung jedoch nicht geeignet, da sie den räumlichen Zustand der Ringstruktur durch die lediglich punktuelle Anbringung nur unzureichend vermitteln können. Außerdem können bekannte Marker die zur Abtragung des Thrombus notwendige Funktionalität, nämlich die Scherkräfte der Vorrichtung, negativ beeinflussen.

Da aber gerade dem proximalen Abschnitt der Thrombektomievorrichtung eine entscheidende Bedeutung bei der erfolgreichen Entfernung des Thrombus zukommt, ist eine möglichst genaue Information über den Öffnungszustand der Ringstruktur während jedes Schrittes der Intervention äußerst wichtig. Denn die erfolgreiche Entfernung des Thrombus aus dem Gefäß hängt wesentlich von einem bestmöglichen Anliegen der Vorrichtung und insbesondere der proximalen Ringstruktur an der Gefäßwand ab, da nur so ein Abschälen des Thrombus von der Gefäßwand erfolgen kann.

Aufgabe der vorliegenden Erfindung ist es somit, eine Thrombektomievorrichtung mit einer röntgendichten Markierung zur Verfügung zu stellen, wobei die röntgendichte Markierung der behandelnden Person eine Beurteilung des dreidimensionalen Zustandes insbesondere der proximalen Ringstruktur während der Intervention ermöglicht, ohne dabei die Funktionalität der Vorrichtung einzuschränken.

Diese Aufgabe wird erfindungsgemäß gelöst durch eine Thrombektomievorrichtung mit einer im Wesentlichen zylindrischen Struktur mit einem proximalen und einem distalen Ende, die eine Vielzahl von Maschen aufweist, welche sich aus Streben aufbauen, wobei die Thrombektomievorrichtung des Weiteren zwei Verbindungsstreben, die an proximalen Maschen am proximalen Ende der zylindrischen Struktur angeordnet sind und sich in Richtung proximal erstrecken, und ein proximal der zylindrischen Struktur angeordnetes Kopplungselement umfasst, mit dem die Verbindungsstreben verbunden sind, wobei das Kopplungselement mit einem Einführdraht verbunden ist, wobei Streben der proximalen Maschen, die Verbindungsstreben und das Kopplungselement sowie ggf. zwischen den proximalen Maschen angeordnete Spannstreben eine geschlossene, ringförmige Struktur ausbilden und die ringförmige Struktur einen Ringmarker mit einem röntgendichten Material zur Kenntlichmachung der räumlichen Lage der ringförmigen Struktur umfasst.

Die erfindungsgemäße Thrombektomievorrichtung umfasst eine im Wesentlichen zylindrische Struktur mit einem proximalen und einem distalen Ende, wobei die zylindrische Struktur von einer Vielzahl von Maschen gebildet wird. Die zylindrische Struktur entspricht im Wesentlichen der eines typischen Stents, allerdings dient die Thrombektomievorrichtung einem anderen Zweck.

Die im Wesentlichen zylindrische Struktur der Thrombektomievorrichtung weist eine Vielzahl von über die Mantelfläche verteilten Öffnungen auf, die erfindungsgemäß als Maschen bezeichnet werden. Mit anderen Worten handelt es sich um eine Gitter- oder Maschenstruktur, aufgebaut aus Streben, sodass sich auf der Mantelfläche der zylindrischen Grundstruktur eine Vielzahl von Öffnungen oder Maschen ergeben. Auch weitere Bereiche der Thrombektomievorrichtung, insbesondere ein sich proximal an die zylindrische Struktur anschließender proximaler Abschnitt, können eine Gitterstruktur mit Öffnungen, aufgebaut aus Streben, aufweisen, wobei der Durchmesser des proximalen Abschnitts in der Regel in Richtung proximal abnimmt, der proximale Abschnitt also nicht zylindrisch ist.

Am proximalen Ende der zylindrischen Struktur sind an (in der Regel verschiedenen) Maschen zwei Verbindungsstreben angeordnet. Die Verbindungsstreben münden in einem Kopplungselement, an das ein Einführdraht zur Platzierung der Vorrichtung angekoppelt ist. Im Bereich des Kopplungselements kann ein proximaler Marker vorgesehen sein. Am distalen Ende der Vorrichtung kann ein distaler Marker vorgesehen sein. Proximaler und distaler Marker können bekannte röntgendichte Materialen umfassen, wie sie nachfolgend noch genannt werden.

Am Kopplungselement oder im Bereich des Einführdrahts kann eine Ablösestelle vorgesehen sein, um bei Bedarf eine Ablösung der zylindrischen Struktur zu erreichen. Da eine Thrombektomievorrichtung jedoch dafür vorgesehen ist, zur Entfernung des Thrombus aus dem Blutgefäßsystem entfernt zu werden, ist eine Ablösestelle nicht unbedingt erforderlich, diese dient vielmehr nur der Ablösung für den Fall, dass sich die Thrombektomievorrichtung nicht zurückziehen lässt. Die Ablösestelle kann beispielsweise ein elektrolytisch korrodierbares System sein, das es erlaubt, die zylindrische Struktur von einem Einführdraht abzulösen. Andere Ablösemechanismen sind dem Fachmann bekannt, beispielsweise mechanische, thermische oder chemische Ablösemechanismen.

Ein Schlitz kann sich optional wendelförmig über die Mantelfläche der zylindrischen Struktur erstrecken. In diesem Fall überspannt eine Spannstrebe, die auch als Spannbügel bezeichnet werden kann, den Schlitz am proximalen Ende der zylindrischen Struktur.

Zumindest einige Streben der proximalen Maschen, die Verbindungsstreben und das Kopplungselement bilden zusammen eine geschlossene Ringstruktur aus. Die ringfömige Struktur wird also von den entsprechenden Streben der proximalen Maschen, den Verbindungsstreben und dem Kopplungselement gebildet.

Sofern die Thrombektomievorrichtung einen Schlitz aufweist, ist auch die Spannstrebe bzw. sind die Spannstreben, der den Schlitz am proximalen Ende der zylindrischen Struktur überspannt, ein Teil der ringförmigen Struktur, d. h. die Spannstrebe, einige Streben der proximalen Maschen, die Verbindungsstreben und das Kopplungselement bilden zusammen die geschlossene ringförmige Struktur aus.

Diese ringförmige Struktur umfasst erfindungsgemäß zumindest einen Ringmarker, der so an der ringförmigen Struktur vorgesehen ist, dass der Marker die räumliche Lage beziehungsweise die räumliche Ausdehnung der ringförmigen Struktur während der Intervention kenntlich machen kann und dem Anwender so eine Beurteilung des Öffnungszustandes der ringförmigen Struktur erlaubt. Der Marker der ringförmigen Struktur wird nachfolgend zur Abgrenzung von weiteren distalen und proximalen Markern als "Ringmarker" bezeichnet. Da die ringförmige Struktur sich normalerweise diagonal durch das Lumen des Blutgefäßes erstreckt, erlaubt der Ringmarker dem behandelnden Arzt auch eine Visualisierung der Orientierung der Thrombektomievorrichtung um die Längsachse.

In einer bevorzugten Ausführungsform umfasst der Ringmarker ein röntgendichtes Material, beispielsweise Gold, Platin, Palladium, Tantal oder entsprechende Kombinationen basierend auf Edelmetallen beziehungsweise mögliche Legierungen und Kombinationen wie beispielsweise Platin-Iridium. Auch für weitere Marker der Thrombektomievorrichtung können diese röntgendichten Materialien verwendet werden.

Vorzugsweise ist der Ringmarker drahtförmig. Weiterhin bevorzugt ist der Ringmarker einteilig vorgesehen, umfasst also einen durchgehenden Draht.

Der Draht an sich kann eine Mehrzahl einzelner Stränge umfassen, die beispielsweise verdreht, verflochten, verdrillt oder in sonstiger Weise verbunden sind. Der Draht kann erfindungsgemäß also auch eine Litze sein. Ein Ringmarker aus einem durchgehenden Draht ist erfindungsgemäß so zu verstehen, dass der Draht beziehungsweise der Ringmarker über seine Länge nicht unterbrochen ist.

Vorzugsweise ist der drahtförmige Ringmarker um die Streben gewunden, die die ringförmige Struktur ausbilden. Alternativ kann der Ringmarker jedoch auch nur auf einer Seite der ringförmigen Struktur, beispielsweise auf der Innen- oder der Außenseite, vorgesehen sein oder die ringförmige Struktur auch nur in einer oder nur wenigen Windungen umwinden.

Sinnvollerweise umläuft der Ringmarker die ringförmige Struktur über ihre gesamte Länge, also ausgehend beispielweise vom Kopplungselement beziehungsweise ausgehend von einem proximalen Marker über die erste Verbindungsstrebe und die Streben der proximalen Maschen hin zur Spannstrebe, sofern vorhanden, und dann zurück über die Streben der proximalen Maschen und die zweite Verbindungsstrebe zum Kopplungselement beziehungsweise zum proximalen Marker.

Die Enden des Ringmarkers sind vorzugsweise im proximalen Marker oder dem Kopplungselement festgelegt.

In alternativen Ausführungsformen kann die Anordnung des Ringmarkers auch auf nur einen oder mehrere Teilbereiche der ringförmigen Struktur begrenzt sein, also beispielsweise auf die Spannstrebe und/oder die Verbindungsstreben und/oder die Streben der proximalen Maschen. Auch in diesem Fall sollte der Ringmarker aber die ringförmige Struktur so weitgehend markieren, dass deren Entfaltung und Orientierung sich aus der Visualisierung mittels entsprechender bildgebender Verfahren im Ganzen ableiten lässt.

Entsprechend ist es auch vorstellbar, dass sich der Ringmarker aus einer Mehrzahl einzelner Ringmarkerelemente zusammensetzt.

Neben der Form eines Drahtes können der Ringmarker und die Ringmarkerelemente auch andere Formen umfassen und beispielsweise spiralförmig, rohrförmig oder hülsenförmig vorgesehen sein.

In weiteren Ausführungsformen ist die zusätzliche Anbringung von Markern entlang der zylindrischen Struktur, beispielsweise im mittleren Bereich, vorgesehen. Auch diese Marker können durchgehend oder zusammengesetzt aus Markerelementen und beispielsweise drahtförmig, spiralförmig, rohrförmig oder hülsenförmig vorgesehen sein.

Der Querschnitt des drahtförmigen Ringmarkers und der weiteren Marker ist vorzugsweise rund, es sind jedoch auch Ausführungsformen mit einem ovalen oder eckigen Draht vorstellbar.

Vorzugsweise weist der Draht einen Durchmesser von 0,01 bis 0,5 mm beziehungsweise eine Kantenlänge von 0,01 bis 0,5 mm auf.

Die Anbringung des Ringmarkers oder der Ringmarkerelemente sowie der Marker oder der Markerelemente erfolgt durch bekannte Methoden, insbesondere durch Schweißen (beispielsweise durch einen Laser), Krimpen, Klemmen oder Kleben.

Neben den genannten Markern ist in einer Weiterbildung der Erfindung eine zusätzliche Markierung von zumindest Teilen der zylindrischen Struktur, also beispielsweise des proximalen, distalen oder mittleren Bereichs, durch eine Beschichtung mit röntgendichten Materialien denkbar. Die für eine Beschichtung geeigneten Materialien entsprechen im Wesentlichen den Materialien, Legierungen und Kombinationen, die zuvor auch für die übrigen Marker als geeignet genannt wurden. Möglich ist beispielsweise eine Goldbeschichtung.

Eine erfindungsgemäße Thrombektomievorrichtung kann auf verschiedene Weise ausgeführt sein. Der dieser Erfindung zugrunde liegende Gedanke der Anbringung eines Ringmarkers entlang der proximalen ringförmigen Struktur ist problemlos auch auf ähnliche Vorrichtungen übertragbar.

Die Spannstrebe kann nachträglich an die zylindrische Struktur angebracht sein, die Spannstrebe kann aber auch Teil der Struktur selbst sein und beispielsweise mit dieser zusammen aus einem Rohr geschnitten werden.

Der proximale Bügel in Form der Spannstrebe verbessert den Radialkraftverlauf der zylindrischen Struktur im proximalen Bereich. Insbesondere vermindert der Bügel eine Verschlankung der zylindrischen Struktur und der Zugbelastung, wie sie beim Einziehen in den Katheter auftritt. Gleichzeitig wird eine zusätzliche Schälwirkung erreicht, wie sie auch von den Maschen und Kanten der zylindrischen Struktur ausgeübt wird.

Von Bedeutung ist aber insbesondere die Verbesserung der Aufspannkraft im proximalen Bereich, die eine optimale Anpassung der zylindrischen Struktur an das Gefäßlumen ermöglicht. Gleichzeitig werden die vom Schlitz voneinander getrennten Bereiche der zylindrischen Struktur daran gehindert, sich gegeneinander zu verschieben.

Um ein problemloses Einziehen der zylindrischen Struktur mit der Spannstrebe in den Katheter zu ermöglichen, ist die Wölbung der Spannstrebe so ausgebildet, dass ihr Maximum zum distalen Ende der zylindrischen Struktur hinweist. Dies bedeutet, dass der Bogen der Spannstrebe nach distal geschlossen ist, dagegen nach proximal zusammen mit den Verbindungsstreben und Streben der proximalen Maschen eine ringförmige Struktur bildet, die im Kopplungselement zusammenläuft.

Alternativ überspannt die Spannstrebe den Schlitz der zylindrischen Struktur in einem wellenförmigen Verlauf, etwa dergestalt, dass die Spannstrebe den Verlauf des Randes der Maschenstruktur auf einer Seite des Schlitzes aufnimmt und zur anderen Seite fortsetzt. Gemäß einer Variante kann die erfindungsgemäße zylindrische Struktur am distalen Ende durch eine Maschenstruktur verschlossen sein, so dass sich thrombotisches Material darin wie in einem Fangkorb sammelt.

Die Maschenstruktur der zylindrischen Struktur kann geflochten sein, d. h. aus einzelnen Drähten bzw. Drahtbündeln als Streben bestehen, ist aber vorzugsweise eine geschnittene Struktur, bei der aus einem Rohr geeigneten Durchmessers mit Hilfe eines Lasers die Maschenstruktur herausgeschnitten wird. Das Material ist in der Regel ein Metall, kann aber auch ein Kunststoff sein. Es muss über eine hinreichende Elastizität verfügen, die eine Kontraktion auf den Durchmesser eines üblichen Katheters und andererseits bei der Freisetzung aus dem Katheter die Expansion auf den gewünschten und vorgegebenen Durchmesser erlaubt. Zusätzlich ist es sinnvoll, die Gitterstruktur einer Elektropolitur zu unterziehen, um sie glatter und abgerundeter und damit weniger traumatisch werden zu lassen. Darüber hinaus sinkt die Gefahr der Anhaftung von Keimen oder sonstigen Verunreinigungen. Die Streben oder Drähte können einen runden, ovalen, quadratischen, rechteckigen oder trapezförmigen Querschnitt aufweisen, wobei im Falle eines quadratischen, rechteckigen oder trapezförmigen Querschnitts eine Abrundung der Kanten von Vorteil ist. Möglich ist auch die Verwendung von flachen Stegen/Drähten in Form dünner Streifen, insbesondere Metallstreifen.

Als Materialien für die Streben kommen neben Eisenlegierungen (Edelstahl, Federstahl) und Kobalt-Chrom-Legierungen insbesondere Formgedächtnislegierungen in Frage, etwa binäre Nickel-Titan-Legierungen (Nitinol) und ternäre Nickel-Titan-Chrom-Legierungen (Chrom-dotierte Legierungen). Insbesondere Nitinol ist für die Anwendung in selbstexpandierenden Strukturen im neurovaskulären Bereich bekannt.

Geeignete Thrombektomievorrichtungen können über die proximale Spannstrebe hinaus weitere Spannbügel im zentralen und distalen Bereich aufweisen. Bei Verwendung von Formgedächtnismaterialien mit hinreichender Vorspannung kann aber auch auf jeglichen Spannbügel verzichtet werden.

Die Thrombektomievorrichtung wird so eingesetzt, dass sie mittels eines Katheters an den Einsatzort verbracht wird und dort entweder im Thrombus selbst, neben oder distal vom Thrombus freigesetzt wird. Die Vorrichtung expandiert im Gefäß und passt sich an das Gefäßlumen an. Entweder schon beim Aufspannen oder beim Zurückziehen verfängt sich das Thrombusmaterial in der Maschenstruktur und wird beim Zurückziehen der Vorrichtung in den Katheter mitgenommen. An der Gefäßwand anhaftende Teile des Thrombus werden durch die Scherwirkung der Maschen und der Kanten entlang des Schlitzes mitgenommen. Der Thrombus wird in den Katheter eingezogen und mit dem Katheter aus dem Körper entfernt.

Durch den Einsatz eines weiteren Katheters, insbesondere eines Aspirationskatheters, können zusätzlich gelöste Teilchen des Thrombus eingesaugt werden, um die Gefahr weiterer durch diese Teilchen ausgelöster Embolien zu minimieren.

Bei der Extraktion des Thrombus hat ein wendelförmiger Verlauf des Schlitzes über die Mantelfläche den besonderen Vorteil, dass die Kanten der zylindrischen Struktur entlang des Schlitzes bei Zug tangential entlang des Umfangs der Gefäßwandung wandern. Dies verbessert die Scherwirkung. Gleichzeitig verbessert (vermindert) sich durch den wendelförmigen Verlauf die Biegesteifigkeit dergestalt, dass eine bessere Anpassung an kurvige Gefäße möglich ist. Dies erleichtert sowohl die Platzierung als auch die Extraktion von Thromben aus komplexen Gefäßstrukturen.

Wie schon festgestellt, wird die erfindungsgemäße zylindrische Struktur vorzugsweise aus einem zylindrischen Rohr mit Hilfe eines Lasers geschnitten. Dies erlaubt es, den einzelnen Maschen einen besonderen Querschnitt zu verleihen, beispielsweise quadratisch, rechteckig oder trapezförmig. Bei den rechteckigen und trapezförmigen Ausführungsformen kann zum einen die schmale Seite des Querschnitts an der Außenfläche liegen, zum anderen die lange Seite. Bevorzugt ist es, dass die schmale Seite sowohl des Rechtecks wie insbesondere des Trapezes zur Gefäßwand weist, was ein leichteres Eindringen des Thrombus in die Maschenstruktur ermöglicht und die gute Verdrängung der Thrombusmasse bei der Expansion der zylindrischen Struktur erlaubt.

Die am proximalen Ende der zylindrischen Struktur angeordneten Verbindungsstreben führen von an den Schlitz angrenzenden proximalen Maschen zu einem Kopplungselement, in dem sie zusammengeführt sind. Sie sind Teile der zylindrischen Struktur und bestehen deshalb in der Regel aus dem gleichen Material.

Die Erfindung wird anhand der Figuren beispielhaft näher erläutert. Es ist darauf hinzuweisen, dass die Figuren bevorzugte Ausführungsvarianten der Erfindung zeigen, die Erfindung ist jedoch nicht hierauf beschränkt. Der Schutzumfang der Erfindung wird durch die Ansprüche definiert. Es zeigen:
- Figur 1: eine räumliche Darstellung der bekannten zylindrischen Struktur;
- Figur 2: eine räumliche Darstellung der bevorzugten Ausführungsform der erfindungsgemäßen Vorrichtung.

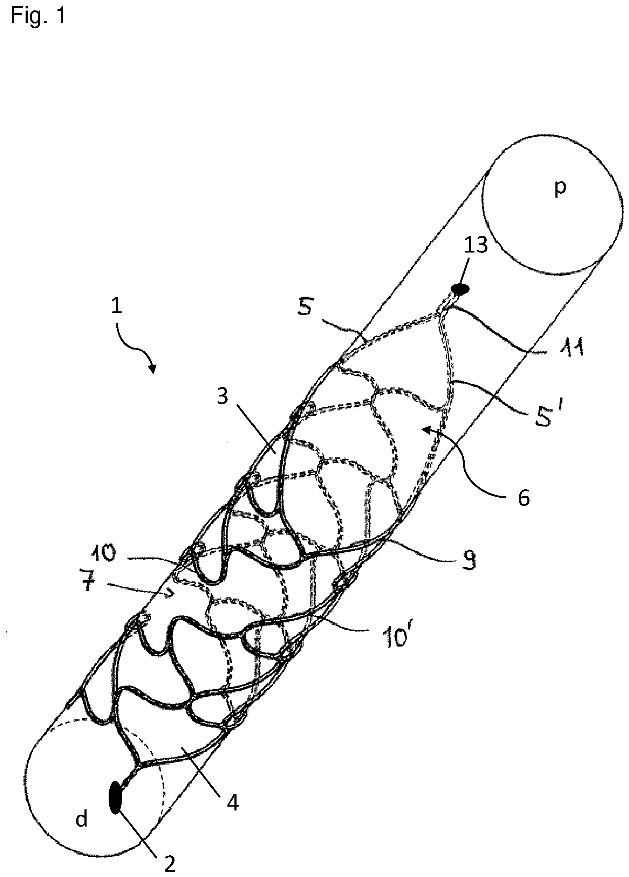

Figur 1 zeigt eine räumliche Darstellung einer Variante der bekannten zylindrischen Struktur 1 mit vorzugweise verschieden ausgestalteten Maschen 3, 4 und den Verbindungsstreben 5 und 5'. Die Maschen 3, 4 geben der Gesamtstruktur Stabilität und Flexibilität.

Durch die zylindrische Struktur 1 verläuft ein Schlitz 7, der am proximalen Ende der zylindrischen Struktur 1 von der Spannstrebe 9 überbrückt wird. Der Schlitz 7 wird begrenzt durch die Ränder 10 und 10' der Maschenstruktur. Der Schlitz 7 verläuft vorzugsweise schräg zur Längsachse der zylindrischen Struktur 1, was sich in der räumlichen Darstellung als wendelförmiger Verlauf entlang der Mantelfläche darstellt.

Die auf der Rückseite der zylindrischen Struktur 1 liegenden Streben der zylindrischen Struktur 1 sind hell dargestellt. Zu erkennen ist der am proximalen Ende der zylindrischen Struktur 1 unter dem Spannbogen 9 durchlaufende Schlitz 7, der sich zur rechten Seite hin um die Mantelfläche der zylindrischen Struktur 1 windet. Der Schlitz 7 endet distal auf der Unterseite der zylindrischen Struktur 1 und beschreibt damit eine Drehung um etwa 180°.

Die Verbindungsstreben 5, 5' sind in einem einzelnen Kopplungselement 11 zusammengeführt, über das eine Verbindung zum hier nur angedeuteten Einführdraht besteht.

Im Bereich des Kopplungselements 11 ist ein proximaler Marker 13 vorgesehen, der das proximale Ende der zylindrischen Struktur 1 angibt.

Der Schlitz 7 wird von der Spannstrebe 9 überbrückt. Die Spannstrebe 9 setzt an den Maschen 3 an, die an den Rändern 10, 10' des Maschenkonstrukts liegen, und weist mit seinem Bogen zur distalen Seite der zylindrischen Struktur 1. Dies erlaubt das problemlose Einziehen der zylindrischen Struktur 1 in einen Katheter.

Die Spannstrebe 9 kann in einer bevorzugten Ausführungsform den Verlauf der Seitenfläche bzw. des Randes 10 der Maschenstruktur mit seinem wellenförmigen Verlauf aufnehmen und setzt diesen zum gegenüberliegenden Rand 10' fort. Die Spannstrebe 9 kann alternativ jedoch auch einen einfach gebogenen, also nicht wellenförmigen Verlauf nehmen.

Die Verbindungsstreben 5, 5' mit den daran anschließenden Maschenrändern und die Spannstrebe 9 bilden insgesamt eine Art Schlaufe, ähnlich der Öffnung eines Fangkorbs, der das Einziehen der Thrombektomievorrichtung in einen Katheter erleichtert und gleichzeitig geeignet ist, an Gefäßwänden anhängende Thromben oder Thrombenreste abzuscheren

Mit dem Kopplungselement 11 zusammen bilden die Verbindungsstreben 5, 5', die daran anschließenden Maschenränder und die Spannstrebe 9 eine ringförmige Struktur.

Das distale Ende der zylindrischen Struktur kann ebenfalls mit einer Maschenstruktur verschlossen sein.

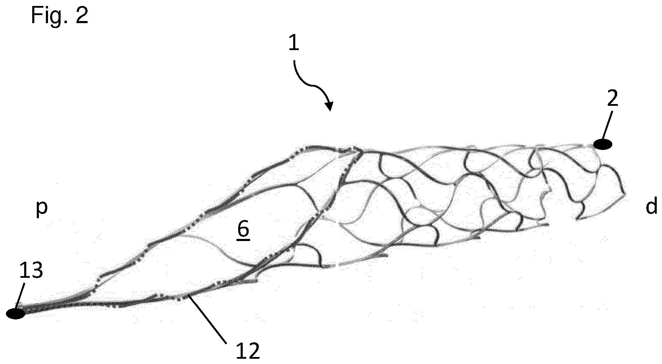

Figur 2 zeigt die erfindungsgemäße Vorrichtung mit einem Ringmarker 12 entlang der proximalen ringförmigen Struktur 6. In der dargestellten bevorzugten Ausführungsform umfasst der Ringmarker 12 ein drahtförmiges röntgendichtes Material, das die ringförmige Struktur 6 über ihren gesamten Umfang schlaufenförmig umwickelt. Vorzugsweise ist der drahtförmige Ringmarker 12 einteilig vorgesehen, umfasst also nur einen einzelnen durchgehenden Draht.

Der Draht an sich kann eine Mehrzahl einzelner Stränge umfassen, die beispielsweise verdreht, verflochten, verdrillt oder in sonstiger Weise verbunden sind. Ein drahtförmiger Ringmarker 12 aus einem durchgehenden Draht ist erfindungsgemäß demnach ein Ringmarker 12, der über seine Länge nicht unterbrochen ist, der also einstückig ist.

Die Enden des Ringmarkers 12 sind im bzw. am proximalen Marker 13 festgelegt.

Weitere proximale Marker 13 und distale Marker 2 können vorgesehen sein.

In den Abbildungen stellen gleiche Bezugszeichen gleiche Sachverhalte dar.

### Bezugszeichenliste

- 1: zylindrische Struktur
- 2: distaler Marker
- 3, 4: Maschen
- 5, 5': Verbindungsstreben
- 6: ringförmige Struktur
- 7: Schlitz
- 9: Spannstrebe
- 10: Ränder der Maschen
- 11: Kopplungselement
- 12: Ringmarker
- 13: proximaler Marker
- d: distal
- p: proximal

## Patentansprüche

1. Thrombektomievorrichtung mit einer im Wesentlichen zylindrischen Struktur (1) mit einem proximalen und einem distalen Ende, die eine Vielzahl von Maschen (3, 4) aufweist, welche sich aus Streben aufbauen, mit
- zwei Verbindungsstreben (5, 5'), die an proximalen Maschen am proximalen Ende der zylindrischen Struktur (1) angeordnet sind und sich in Richtung proximal erstrecken, und
- einem proximal der zylindrischen Struktur (1) angeordneten Kopplungselement (11), mit dem die Verbindungsstreben (5, 5') verbunden sind, wobei das Kopplungselement (11) mit einem Einführdraht verbunden ist,
wobei Streben der proximalen Maschen, die Verbindungsstreben (5, 5') und das Kopplungselement (11) sowie ggf. zwischen den proximalen Maschen angeordnete Spannstreben (9) eine geschlossene, ringförmige Struktur (6) ausbilden,
**dadurch gekennzeichnet, dass**
die ringförmige Struktur (6) einen Ringmarker (12) mit einem röntgendichten Material zur Kenntlichmachung der räumlichen Lage der ringförmigen Struktur (6) umfasst und der Ringmarker (12) drahtförmig oder hülsenförmig und um die die ringförmige Struktur (6) ausbildenden Streben gewunden oder gelegt ist.

2. Thrombektomievorrichtung nach Anspruch 1, **gekennzeichnet durch** einen Schlitz (7), der sich wendelförmig über die Mantelfläche der zylindrischen Struktur (1) erstreckt, wobei eine Spannstrebe (9) am proximalen Ende der zylindrischen Struktur (1) den Schlitz (7) überspannt.

3. Thrombektomievorrichtung nach Anspruch 1 oder 2, **dadurch gekennzeichnet, dass** der Ringmarker (12) die ringförmige Struktur (6) in ihrer Länge zumindest teilweise umfasst.

4. Thrombektomievorrichtung nach Anspruch 3, **dadurch gekennzeichnet, dass** der Ringmarker (12) die ringförmige Struktur (6) über ihre gesamte Länge umfasst.

5. Thrombektomievorrichtung nach Anspruch 1, **dadurch gekennzeichnet, dass** der drahtförmige Ringmarker (12) einstückig ist.

6. Thrombektomievorrichtung nach einem der Ansprüche 1 bis 5, **gekennzeichnet durch** einen proximalen Marker (13) im Bereich des Kopplungselements (11).

7. Thrombektomievorrichtung nach einem der Ansprüche 1 bis 6, **dadurch gekennzeichnet, dass** die Enden des drahtförmigen Ringmarkers (12) im oder am proximalen Marker (13) oder Kopplungselement (11) festgelegt sind.

8. Thrombektomievorrichtung einem der Ansprüche 1 bis 7, **dadurch gekennzeichnet, dass** die Spannstrebe (9) eine Wölbung ausbildet, wobei das Maximum der Wölbung zum distalen Ende der zylindrischen Struktur (1) weist.

9. Thrombektomievorrichtung nach einem der Ansprüche 1 bis 8, **gekennzeichnet durch** weitere distale und/oder proximale Marker aus einem röntgendichten Material.

10. Thrombektomievorrichtung nach einem der Ansprüche 1 bis 9, **dadurch gekennzeichnet, dass** das röntgendichte Material ausgewählt ist aus Gold, Tantal, Platin, Palladium oder einer Legierung basierend auf Edelmetallen oder einer Kombination dieser Materialien wie Platin-Iridium.

11. Thrombektomievorrichtung nach einem der Ansprüche 1 bis 10, **dadurch gekennzeichnet, dass** weitere Marker entlang der zylindrischen Struktur (1) vorgesehen sind, wobei diese Marker drahtförmig, spiralförmig, rohrförmig oder hülsenförmig vorgesehen sein können.

12. Thrombektomievorrichtung nach einem der Ansprüche 1 bis 11, **dadurch gekennzeichnet, dass** eine zusätzliche Markierung von zumindest Teilen der zylindrischen Struktur (1) durch eine Beschichtung mit röntgendichten Materialien vorgesehen ist.

## Claims

1. Thrombectomy device comprising an essentially cylindrical structure (1) having a proximal end and a distal end and comprising a plurality of meshes (3, 4) composed of struts, including
- two connecting struts (5, 5') arranged at proximal meshes at the proximal end of the cylindrical structure (1) and extending in the proximal direction, and
- a coupling member (11) arranged proximally of the cylindrical structure (1), with connecting struts (5, 5') being attached to said member, and said coupling member (11) being connected to an insertion wire,
wherein struts of the proximal meshes, the connecting struts (5, 5') and the coupling member (11) as well as bracing struts (9) possibly arranged between the proximal meshes form a closed, annular structure (6),
**characterized in that**
the annular structure (6) comprises a ring marker (12) with a radiopaque material for visualizing the spatial position of the annular structure (6), and the ring marker (12) is wire-shaped or sleeve-shaped and is wound or laid around the struts forming the annular shaped structure (6).

2. Thrombectomy device according to Claim 1, **characterized by** a slit (7) extending in a helical fashion over the circumferential surface of the cylindrical structure (1), with a bracing strut (9) spanning the slit (7) at the proximal end of the cylindrical structure (1).

3. Thrombectomy device according to Claim 1 or 2, **characterized in that** the ring marker (12) encompasses the annular structure (6) at least partially along its length.

4. Thrombectomy device according to Claim 3, **characterized in that** the ring marker (12) encompasses the annular structure (6) along its entire length.

5. Thrombectomy device according to Claim 1, **characterized in that** the wire-shaped ring marker (12) is of one-piece design.

6. Thrombectomy device according to any one of Claims 1 to 5, **characterized by** a proximal marker (13) in the region of the coupling member (11).

7. Thrombectomy device according to any one of Claims 1 to 6, **characterized in that** the ends of the wire-shaped ring marker (12) are secured in or to the proximal marker (13) or coupling member (11).

8. Thrombectomy device according to any one of Claims 1 to 7, **characterized in that** the bracing strut (9) forms a camber, with the maximum of the camber pointing toward the distal end of the cylindrical structure (1).

9. Thrombectomy device according to any one of Claims 1 to 8, **characterized by** further distal and/or proximal markers made of a radiopaque material.

10. Thrombectomy device according to any one of Claims 1 to 9, **characterized in that** the radiopaque material is selected from gold, tantalum, platinum, palladium or an alloy based on precious metals or a combination of these materials such as platinum-iridium.

11. Thrombectomy device according to any one of Claims 1 to 10, **characterized in that** further markers are provided along the cylindrical structure (1), and these markers may be provided in wire form, spiral form, tubular form, or sleeve form.

12. Thrombectomy device according to any one of Claims 1 to 11, **characterized in that** an additional marking of at least parts of the cylindrical structure (1) is provided by coating with radiopaque materials.

## Revendications

1. Dispositif de thrombectomie comprenant une structure sensiblement cylindrique (1) avec une extrémité proximale et une extrémité distale, qui présente une pluralité de mailles (3, 4) constituées de montants, avec
- deux montants de liaison (5, 5') qui sont disposées sur des mailles proximales à l'extrémité proximale de la structure cylindrique (1) et s'étendent dans la direction proximale, et
- un élément de couplage (11) disposé à proximité de la structure cylindrique (1), auquel sont reliées les montants de liaison (5, 5'), dans lequel l'élément de couplage (11) étant relié à un fil d'introduction,
dans lequel les montants des mailles proximales, les montants de liaison (5, 5') et l'élément de couplage (11) ainsi que, le cas échéant, des montants de tension (9) disposées entre les mailles proximales formant une structure annulaire fermée (6),
**caractérisé en ce que**
la structure annulaire (6) comprend un marqueur annulaire (12) avec un matériau radio-opaque pour identifier la position spatiale de la structure annulaire (6) et le marqueur annulaire (12) est en forme de fil ou de manchon et est enroulé ou posé autour des entretoises formant la structure annulaire.

2. Dispositif de thrombectomie selon la revendication 1, **caractérisé par** une fente (7) qui s'étend en spirale sur la surface enveloppe de la structure cylindrique (1), dans lequel un montant de tension (9) à l'extrémité proximale de la structure cylindrique (1) enjambant la fente (7).

3. Dispositif de thrombectomie selon la revendication 1 ou 2, **caractérisé en ce que** le marqueur annulaire (12) entoure au moins partiellement la structure annulaire (6) sur toute sa longueur.

4. Dispositif de thrombectomie selon la revendication 3, **caractérisé en ce que** le marqueur annulaire (12) entoure la structure annulaire (6) sur toute sa longueur.

5. Dispositif de thrombectomie selon la revendication 1, **caractérisé en ce que** le marqueur annulaire en forme de fil (12) est d'un seul tenant.

6. Dispositif de thrombectomie selon l'une des revendications 1 à 5, **caractérisé par** un marqueur proximal (13) dans la zone de l'élément de couplage.

7. Dispositif de thrombectomie selon l'une des revendications 1 à 6, **caractérisé en ce que** les extrémités du marqueur annulaire en forme de fil (12) sont fixées dans ou sur le marqueur proximal (13) ou l'élément de couplage (11).

8. Dispositif de thrombectomie selon l'une des revendications 1 à 7, **caractérisé en ce que** le montant de tension (9) forme une courbure, le maximum de la courbure étant orienté vers l'extrémité distale de la structure distale.

9. Dispositif de thrombectomie selon l'une des revendications 1 à 8, **caractérisé par** d'autres marqueurs distaux et/ou proximaux en un matériau radio-opaque.

10. Dispositif de thrombectomie selon l'une des revendications 1 à 9, **caractérisé en ce que** le matériau radio-opaque est choisi parmi l'or, le tantale, le platine, le palladium ou un alliage à base de métaux précieux ou une combinaison de ces matériaux, comme le platine-iridium.

11. Dispositif de thrombectomie selon l'une des revendications 1 à 10, **caractérisé en ce que** d'autres marqueurs sont prévus le long de la structure cylindrique (1), ces marqueurs pouvant être en forme de fil, de spirale, de tube ou de manchon.

12. Dispositif de thrombectomie selon l'une des revendications 1 à 11, **caractérisé en ce qu'**un marquage supplémentaire d'au moins certaines parties de la structure cylindrique (1) est prévu par un revêtement avec des matériaux radio-opaques.
